# EUROPEAN PATENT APPLICATION

(11) **EP 3 275 490 A1**
(43) Date of publication of application: **31.01.2018**
(21) Application number: 16772027.5
(22) Date of filing: 29.02.2016
(51) Int. Cl.: A61M 5/32

(54) **INJECTION NEEDLE ASSEMBLY, AND DRUG INJECTION DEVICE**

(30) Priority: 27.03.2015 JP 2015066424
(71) Applicant: Terumo Kabushiki Kaisha, Tokyo 151-0072 (JP)
(72) Inventor: KAWAMOTO Eiji, Ashigarakami-gun Kanagawa 259-0151 (JP)
(74) Representative: Casalonga
(86) International application number: PCT/JP2016/055999
(87) International publication number: WO 2016/158143

(57) **Abstract**

An injection needle assembly includes a needle tube, a needle hub, a main body portion, a connecting portion, and an energizing portion. The main body portion can move to a first position which is away from the needle tip of the needle tube and where the needle tip of the needle tube is exposed and a second position where the main body portion covers the needle tip of the needle tube. In the first position, at least a part of the main body portion protrudes to the needle tip side of the needle tube relative to a contact surface.

## Description

### Technical Field

The present invention relates to an injection needle assembly and a drug injection device used when a drug is injected into a living body.

### Background Art

The drug injection device includes an injection needle assembly having a needle tube and a syringat contains a drug. The needle tube included in the injection needle assembly has a needle tip that can puncture a living body. The needle tip of the needle tube is exposed at all times, so that there is a risk that the needle tip of the needle tube erroneously punctures a user after giving a drug or when disposing of the drug injection device.

To solve such a problem, for example, Patent Literature 1 describes a technique that includes a rotatable protector main body and a rotation support portion. In the technique described in Patent Literature 1, after the needle tube punctures a living body, the protector main body is rotated and the needle tip of the needle tube is covered by the protector main body. Thereby, a used needle tip of the needle tube is prevented from erroneously puncturing a user.

### Citation List

### Patent Literature

Patent Literature 1: WO 2013/046857 A

### Summary of Invention

### Technical Problem

However, in the technique described in Patent Literature 1, a rotation operation of the protector main body is performed by hands of a user. Therefore, there is not only a risk that the user forgets to perform the rotation operation of the protector main body, but also a risk that a finger or the like of the user is erroneously punctured by the needle tip of the needle tube when the user rotates the protector main body.

Considering the problems described above, an object of the present invention is to provide an injection needle assembly and a drug injection device which can reliably cover a used needle tip of the needle tube to improve safety.

### Solution to Problem

To solve the above problem and achieve the object of the present invention, the injection needle assembly of the present invention includes a needle tube, a needle hub, amain body portion, a connecting portion, and an energizing portion. The needle tube has a needle tip that can puncture a living body. The needle hub holds the needle tube and has a contact surface that comes into contact with a living body when the needle tip of the needle tube is punctured into the living body. The main body portion can move to a first position which is away from the needle tip of the needle tube and where the needle tip of the needle tube is exposed and a second position where the main body portion covers the needle tip of the needle tube. The connecting portion rotatably connects the main body portion to the needle hub. The energizing portion is provided to the main body portion and energizes the main body portion from the first position toward the second position when being elastically deformed. In the first position, at least a part of the main body portion protrudes to the needle tip side of the needle tube relative to the contact surface.

The drug injection device of the present invention includes an injection needle assembly and a syringe that is detachably attached to the injection needle assembly. As the injection needle assembly, the aforementioned injection needle assembly is used.

### Advantageous Effects of Invention

According to the injection needle assembly and the drug injection device of the present invention, it is possible to reliably cover a used needle tip of the needle tube with the main body portion and it is possible to prevent the used needle tip of the needle tube from erroneously puncturing a user. Therefore, safety can be improved.

### Brief Description of Drawings

Fig. 1 is a perspective view illustrating a drug injection device according to a first embodiment of the present invention.
Fig. 2 is a cross-sectional view illustrating an injection needle assembly of the drug injection device according to the first embodiment of the present invention.
Fig. 3 is a cross-sectional view illustrating a state of the drug injection device according to the first embodiment of the present invention during puncture.
Fig. 4 is a cross-sectional view illustrating a state of the drug injection device according to the first embodiment of the present invention after puncture.
Fig. 5 is a cross-sectional view illustrating a state of the drug injection device according to the first embodiment of the present invention after puncture.
Fig. 6 is a cross-sectional view illustrating a drug injection device according to a second embodiment of the present invention.
Fig. 7 is a cross-sectional view illustrating a drug injection device according to a third embodiment of the present invention.

### Description of Embodiments

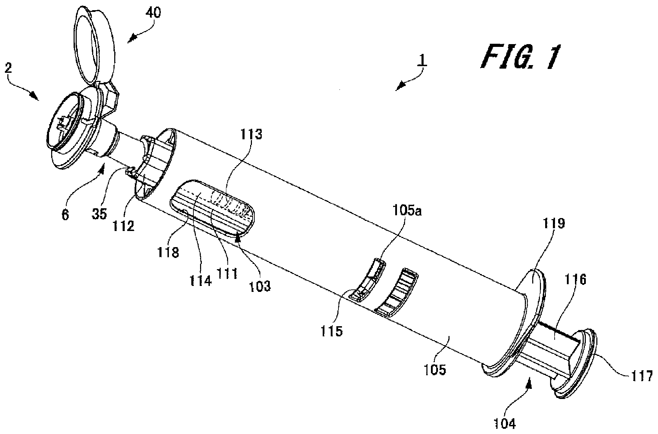

Hereinafter, embodiments of the injection needle assembly and the drug injection device of the present invention will be described with reference to Figs. 1 to 7. In the drawings, the same members are denoted by the same reference numerals. The present invention is not limited to the embodiments described below.

The embodiments will be described in the following order.
1. First Embodiment
1-1. Configuration example of injection needle assembly and drug injection device
1-2. Method of using drug injection device
2. Second Embodiment
3. Third Embodiment

### 1. First Embodiment

### 1-1. Configuration example of injection needle assembly and drug injection device

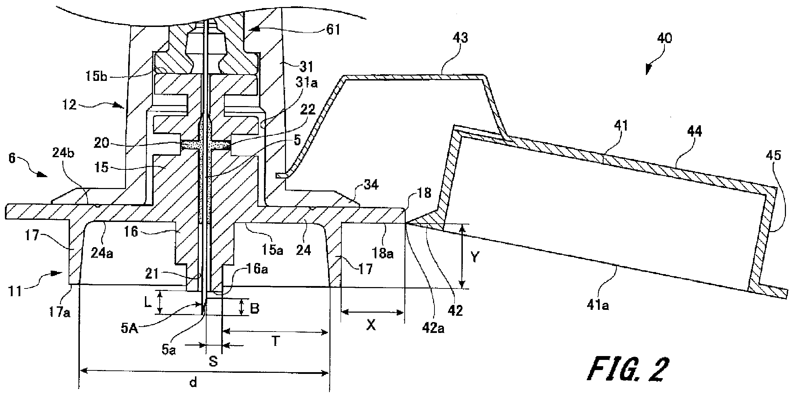

First, a drug injection device and an injection needle assembly according to the first embodiment of the present invention (hereinafter referred to as "the present embodiment") will be described with reference to Figs. 1 and 2.

Fig. 1 is a perspective view illustrating a drug injection device of the present embodiment. Fig. 2 is a cross-sectional view illustrating an injection needle assembly of the present embodiment.

The drug injection device 1 is used when puncturing a skin surface with a needle tip and injecting drug into a skin upper layer region.

Here, the skin is composed of three portions: the epidermis, the dermis, and the subcutaneous tissue. The epidermis is a layer of about 50 to 200 µm ranging from the surface of the skin, and the dermis is a layer of about 1.5 to 3.5 mm continuing from the epidermis. An influenza vaccine is ordinarily administered subcutaneously or intramuscularly, and, therefore, it is injected into a lower layer portion of the skin or a portion deeper than the lower layer portion.

To reduce doses of the influenza vaccine, administration of the vaccine while taking as a target region a skin upper layer region where a lot of immunocytes are present has been investigated. The term "skin upper layer region" used here means the epidermis and the dermis, of the skin. The drug injection device 1 of the present embodiment is the drug injection device 1 for intradermal injection whose target region is such a skin upper layer region.

As illustrated in Fig. 1, the drug injection device 1 has an injection needle assembly 2, a syringe 103 that is detachably attached to the injection needle assembly 2, a pusher member 104, and a syringe holder 105 that holds the syringe 103.

### [Syringe]

The syringe 103 is a pre-filled syringe that is filled with a drug in advance. The syringe 103 has a syringe main body 111, a discharge portion that is formed at one end portion in an axial direction of the syringe main body 111, a lock mechanism 112 provided to the discharge portion, and a gasket 113.

The syringe main body 111 is formed into a hollow and substantially cylindrical shape. The gasket 113 is slidably arranged inside a cylindrical hole of the syringe main body 111. The gasket 113 is formed into a substantially columnar shape and is closely and liquid-tightly attached to an inner circumferential surface of the cylindrical hole of the syringe main body 111. The gasket 113 partitions an internal space of the syringe main body 111 into two. A space from the gasket 113 toward the discharge portion in the syringe main body 111 is a liquid chamber 114 that is filled with a drug. On the other hand, in a space from the gasket 113 toward the other end in the syringe main body 111, a plunger main body 116 of the pusher member 104 described later is inserted.

Though a material of the gasket 113 is not limited in particular, it is preferable that the gasket 113 is formed of an elastic material to achieve good liquid-tightness with the syringe main body 111. Examples of the elastic material include various rubber materials such as natural rubber, isobutylene rubber, and silicone rubber, various thermoplastic elastomers such as olefinic elastomer and styrene elastomer, and a mixture thereof and the like.

The outer and inner diameters of the syringe main body 111 are appropriately set according to purpose of use of the drug injection device 1 and the amount of drug contained in the liquid chamber 114. For example, when the amount of drug contained by using a general-purpose high-speed filling machine is 0.5 mL, it is preferable to set the inner diameter of the syringe main body 111 to 4.4 to 5.0 mm and set the outer diameter of the syringe main body 111 to 6.5 to 8.4 mm. Further, when the amount of drug is 1 mL, it is preferable to set the inner diameter of the syringe main body 111 to 6.1 to 9.0 mm and set the outer diameter of the syringe main body 111 to 7.9 to 12.5 mm.

Examples of the drug include various vaccines that prevent various infectious diseases such as influenza. However, the drug is not limited to vaccines. Examples of the drug other than vaccines include carbohydrate injection solution such as glucose, electrolyte correction injection solution such as sodium chloride, potassium lactate, and the like, vitamin preparations, antibiotic injection solution, imaging agent, steroid drug, proteolytic enzyme inhibitor, fat emulsion, anticancer drug, anesthetics, heparin calcium, and antibody drug.

A flange portion 115 is formed at the other end portion in the axial direction of the syringe main body 111. The flange portion 115 is engaged with an engaging portion 105a provided to the syringe holder 105 described later. The discharge portion not illustrated in the drawings is continuously formed at the one end portion in the axial direction of the syringe main body 111.

The discharge portion is formed into a substantially cylindrical shape coaxial with the syringe main body 111. A cylindrical hole of the discharge portion communicates with the cylindrical hole of the syringe main body 111. The discharge portion is formed into a tapered shape whose diameter is continuously reduced toward one end in an axial direction. When the injection needle assembly 2 is attached to the syringe 103, a distal end portion of the discharge portion liquid-tightly comes into contact with an end face of an elastic member 61 of the injection needle assembly 2 described later.

The lockmechanism 112 is provided to the discharge portion. The lock mechanism 112 is a luer lock unit representing an example of a fixing mechanism. The lock mechanism 112 is formed into a tubular shape that coaxially surrounds the discharge portion. The lock mechanism 112 is formed into a shape whose inner circumference has a circular shape and whose outer circumference has a hexagonal shape. A female screw portion is formed on an inner circumferential surface of the lock mechanism 112. The female screw portion is formed so as to be able to be screwed with a male screw portion 35 provided on the injection needle assembly 2.

Examples of a material of the syringe main body 111 include various resins such as polyvinyl chloride, polyethylene, polypropylene, cyclic polyolefin, polystyrene, poly-(4-methylpentene-1), polycarbonate, acrylic resin, acrylonitrile-butadiene-styrene copolymer, polyester including polyethylene terephthalate, butadiene-styrene copolymer, and polyamide (for example, nylon 6, nylon 6, 6, nylon 6, 10, and nylon 12). Among them, it is preferable to use resins such as polypropylene, cyclic polyolefin, polyester, and poly-(4-methylpentene-1) because they are easily formed. The material of the syringe main body 111 is preferred to be substantially transparent to secure internal visibility.

In the present embodiment, an example has been described where a pre-filled syringe that is filled with a drug in advance is applied as the syringe 103. However, the syringe is not limited to this, and a syringe whose syringe main body is not filled with a drug in advance may be applied.

### [Pusher Member]

The pusher member 104 has a plunger main body 116 and an operation portion 117 that operates the plunger main body 116. The plunger main body 116 is formed into a rod shape. The plunger main body 116 is inserted into the cylindrical hole of the syringe main body 111 through an opening portion formed at the other end portion in the axial direction of the syringe main body 111. Then, one end portion in the axial direction of the plunger main body 116 comes into contact with the gasket 113.

The operation portion 117 is formed at the other end portion in the axial direction of the plunger main body 116. The operation portion 117 is formed into a substantially disk shape. When the drug injection device 1 is used, the operation portion 117 is pressed by a user. Thereby, the one end portion in the axial direction of the plunger main body 116 comes into contact with the gasket 113 to cause the gasket 113 to slide toward the discharge portion.

As a material of the pusher member 104, the various resins included as the examples of a material of the syringe main body 111 can be used.

### [Syringe Holder]

Next, the syringe holder 105 will be described.

The syringe holder 105 is formed into a substantially cylindrical shape. The syringe holder 105 covers the outer circumferential surface of the syringe main body 111 and the outer circumferential surface of the lock mechanism 112 in the syringe 103. The syringe holder 105 is formed so as to be held by a user when the user attaches the injection needle assembly 2 to the syringe 103.

A view window 118 is formed at one end portion in the axial direction of the syringe holder 105. The view window 118 is provided at a position from which the liquid chamber 114 of the syringe 103 can be viewed from the outside of the syringe holder 105 when the syringe 103 is inserted into the syringe holder 105. Thereby, it is possible to secure internal visibility even when the syringe holder 105 is attached to the syringe 103.

A holder brim portion 119 is formed at the other end in the axial direction of the syringe holder 105. The holder brim portion 119 projects substantially perpendicularly from a part of the outer circumferential surface of the syringe holder 105. The holder brimportion 119 is provided, and thereby it is possible to prevent fingers that hold the syringe holder 105 from slipping toward the other end portion in the axial direction when the user holds the syringe holder 105 and administers a drug. Further, when the drug injection device 1 is placed on a desk, a table, or the like, it is possible to prevent the drug injection device 1 from rolling.

Further, an engaging portion 105a is provided in a middle portion in the axial direction of the syringe holder 105. The engaging portion 105a is an opening portion that penetrates an outer wall of the syringe holder 105. The flange portion 115 of the syringe 103 is engaged with the engaging portion 105a.

When the syringe holder 105 is attached to the syringe 103, the diameter of the drug injection device 1 can be increased, so that it is possible to easily hold the drug injection device 1. Thereby, operability when operating the pusher member 104 is improved.

### [Injection Needle Assembly]

Next, the injection needle assembly 2 will be described.

As illustrated in Figs. 1 and 2, the injection needle assembly 2 has a hollow needle tube 5 having a needle hole, a needle hub 6 to which the needle tube 5 is fixed, and a lid member 40 that covers the needle tube 5 after puncture.

As illustrated in Fig. 2, as the needle tube 5, a needle tube having a size of 26 to 33 gauge (outer diameter is 0.2 to 0.45 mm) in ISO standard for medical needle tubes (ISO9626: 1991/Amd. 1:2001(E)) is used, and preferably a needle tube having a size of 30 to 33 gauge is used. A needle smaller than 33 gauge may also be used.

A needle tip 5A having a blade surface 5a is provided at one end of the needle tube 5. Hereinafter, a portion located opposite to the needle tip 5A is referred to as a proximal end of the needle tube 5. The length in the axial direction of the needle tube 5 on the blade surface 5a (hereinafter referred to as a "bevel length B") may be smaller than or equal to 1.4 mm (adults), which is the smallest thickness of a skin upper layer region described later, and may be greater than or equal to 0.5 mm, which is a bevel length when a short bevel is formed on a needle tube of 33 gauge. In short, the bevel length B is preferably set in a range of 0.5 to 1.4 mm.

Further, it is more preferable that the smallest thickness of the skin upper layer region is smaller than or equal to 0.9 mm (infants), that is, the bevel length B is in a range of 0.5 to 0.9 mm. The short bevel means a blade surface that forms an angle of 18 to 25° with respect to the longitudinal direction of the needle, which is generally used for an injection needle.

The material of the needle tube 5 may be, for example, stainless steel, but this is not limitative. Examples of other usable materials include such metals as aluminum, aluminum alloys, titanium, and titanium alloys. In addition, the needle tube 5 is not limited to a straight needle but may be a tapered needle which is tapered at least along a portion of its length. The tapered needle may be one in which the diameter of the proximal end portion of the needle is greater than the distal end portion, with an intermediate part having a tapered structure. The cross-sectional shape of the needle tube 5 is not limited to a circle but may be a polygon such as a triangle.

Next, the needle hub 6 will be described.

The needle hub 6 includes a first member 11 that holds the needle tube 5, a second member 12 into which the discharge portion of the syringe 103 is fitted, and an elastic member 61. Although the first member 11 and the second member 12 are formed as separate members, they can be integrally formed. Examples of material (s) of the first member 11 and the second member 12 include synthetic resins such as polycarbonate, polypropylene, polyethylene, and the like.

The first member 11 includes a base portion 15, an adjustment portion 16, a stabilization portion 17, and a guide portion 18. The base portion 15 is formed into a substantially columnar shape, and has end faces 15a and 15b perpendicular to the axial direction. The adjustment portion 16 is provided at a central portion of the end face 15a of the base portion 15, and is composed of a columnar projected portion projecting in the axial direction of the base portion 15. The axial center of the adjustment portion 16 coincides with the axial center of the base portion 15.

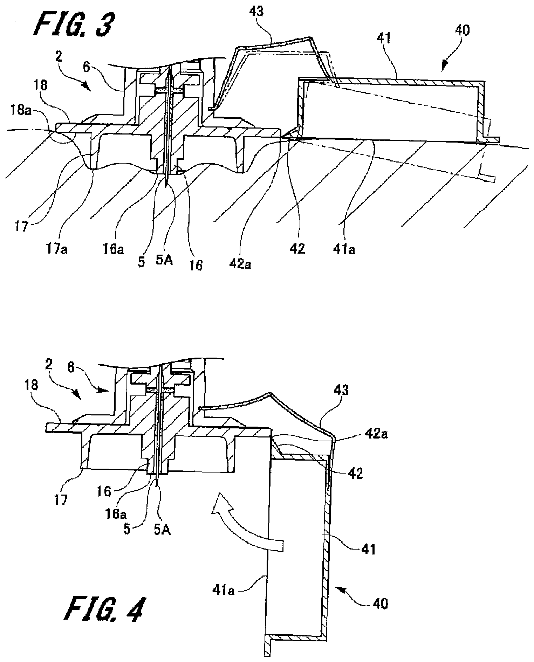

A through-hole 21 through which the needle tube 5 penetrates is provided at the axial center of the base portion 15 and the adjustment portion 16. The base portion 15 includes an injection hole 22 (see Figs. 2 and 4) for injecting an adhesive 20 (see Fig. 3) into the through-hole 21. The injection hole 22 opens to the outer circumferential surface of the base portion 15, and communicates with the through-hole 21. By virtue of the adhesive 20 injected through the injection hole 22 into the through-hole 21, the needle tube 5 is fixed to the base portion 15.

The proximal end of the needle tube 5 protrudes from the end face 15b of the base portion 15. The base portion 15 is inserted into the second member 12 from the end face 15b, and the proximal end of the needle tube 5 is inserted into an insertion hole of the elastic member 61. Then, the end face 15b of the base portion 15 comes into contact with the elastic member 61.

The outer circumferential surface of the base portion 15 includes a connectionpiece 24. The connectionpiece 24 is formed as a ring-shaped flange projecting in the radial directions of the base portion 15, and has flat surfaces 24a and 24b which face opposite to each other in the axial direction of the base portion 15. The secondmember 12 is connected to the flat surface 24b of the connection piece 24. In addition, a tip portion of the connection piece 24 constitutes a guide portion 18. The guide portion 18 will be described in detail later.

An end face of the adjustment portion 16 constitutes a needle protrusion surface 16a from which the needle tip 5A of the needle tube 5 protrudes. The needle protrusion surface 16a is formed as a flat surface orthogonal to the axial direction of the needle tube 5. When the needle tube 5 is positioned to puncture the skin upper layer region, the needle protrusion surface 16a contacts the surface of the skin to determine a puncture depth of the needle tube 5. Specifically, the depth of puncture of the needle tube 5 into the skin upper layer region is determined by the length of a portion of the needle tube 5 which protrudes from the needle protrusion surface 16a (this length will hereinafter be referred to as a "protrusion length L").

The thickness of the skin upper layer region corresponds to a depth ranging from the surface of the skin to the dermis layer, and it is in a range of approximately 0.5 to 3.0 mm. Therefore, the protrusion length L of the needle tube 5 can be set in a range of 0.5 to 3.0 mm.

Vaccines are generally administered into an upper arm region, and, in the case of administration into a skin upper layer region, the target site is preferably a thick-skinned shoulder peripheral region, particularly, a deltoid region. In view of this, the thickness of the skin upper layer region at the deltoid muscle was measured, for 19 infants and 31 adults. The measurement was conducted by imaging the skin upper layer region where ultrasonic reflectance is high, by use of an ultrasonic measuring instrument (NP60R-UBM, a high-resolution echo system for small animals, produced by NEPA GENE CO. , LTD.). The measurements were in log normal distribution and, therefore, the range of MEAN ± 2SD was determined by use of geometric mean.

As a result, the thickness of the skin upper layer region at the deltoid muscles of infants was found to be 0.9 to 1.6 mm. The thickness of the skin upper layer region at the deltoid muscles of adults was found to be 1.4 to 2.6 mm in a distal region, 1.4 to 2. 5 mm in a central region, and 1. 5 to 2.5 mm in a proximal region. From these measurements, it was confirmed that the thickness of the skin upper layer region at the deltoid muscle is not less than 0.9 mm in infants, and not less than 1.4 mm in adults. Accordingly, it is preferable that the protrusion length L of the needle tube 5, in injection into the skin upper layer region at the deltoid muscle, is set in a range of 0.9 to 1.4 mm.

By setting the protrusion length L in this manner, the blade surface 5a of the needle tip 5A can be securely positioned in the skin upper layer region. As a result, a needle hole (medicine discharge port) opening at the blade surface 5a can be located in the skin upper layer region, irrespective of the position of the needle hole on the blade surface 5a. Even when the medicine discharge port is located in the skin upper layer region, the medicine will flow into a subcutaneous region through a gap between a side surface of an end portion of the needle tip 5A and an incised skin if the needle tip 5A pierces to a position deeper than the skin upper layer region. Therefore, it is important that the blade surface 5a is securely located within the skin upper layer region.

In the case of a needle tube thicker than 26 gauge, it is difficult to set the bevel length B to be not more than 1.0 mm. In order to set the protrusion length L of the needle tube 5 to be within the preferable range (0.9 to 1.4 mm), therefore, it is preferable to use a needle tube thinner than 26 gauge.

The needle protrusion surface 16a is so formed that a distance S from its circumferential edge to the outer circumferential surface of the needle tube 5 is not more than 1.4 mm, preferably in a range of 0.3 to 1.4 mm. This distance S from the circumferential edge of the needle protrusion surface 16a to the outer circumferential surface of the needle tube 5 is determined by taking into account that a pressure is exerted on a wheal or blister formed by injection of the medicine into the skin upper layer region. Specifically, the needle protrusion surface 16a is set to be sufficiently smaller than the wheal or blister to be formed in the skin upper layer region and, hence, does not inhibit the formation of the wheal or blister. As a result, even when the needle protrusion surface 16a presses against the skin in the surroundings of the needle tube 5, the medicine being injected can be prevented from leaking.

The stabilization portion 17 is formed into a tubular shape that protrudes from the flat surface 24a of the connection piece 24 provided on the base portion 15. The needle tube 5 and the adjustment portion 16 are disposed in a cylindrical hole of the stabilization portion 17. In other words, the stabilization portion 17 is formed into a cylindrical shape that covers the surrounding of the adj ustment portion 16 penetrated by the needle tube 5, and is provided radially separated from the needle tip 5A of the needle tube 5.

As shown in Fig. 3, an end face 17a of the stabilization portion 17 is located on the proximal end side of the needle tube 5 relative to the needle protrusion surface 16a of the adjustment portion 16. When the needle tip 5A of the needle tube 5 punctures a living body, the needle protrusion surface 16a first comes into contact with the surface of the skin, and thereafter the surface of the skin touches the end face 17a of the stabilization portion 17. In this instance, the contact of the end face 17a of the stabilization portion 17 with the skin helps stabilize the drug injection device 1, whereby the needle tube 5 can be kept in a posture of being substantially perpendicular to the skin.

When the end face 17a of the stabilization portion 17 is located on the same plane as the needle protrusion surface 16a, or is located on the side of the needle tip 5A of the needle tube 5 relative to the needle protrusion surface 16a, the needle tube 5 can be maintained in a posture of being substantially perpendicular to the skin. Taking into account the bulging of the skin when the stabilization portion 17 is pressed against the skin, the distance between the end face 17a of the stabilization portion 17 and the needle protrusion surface 16a along the axial direction is preferably set to be not more than 1.3 mm.

In addition, an inside diameter d of the stabilization portion 17 is set to a value equal to or greater than the diameter of the wheal or blister to be formed in the skin. Specifically, the inside diameter d of the stabilization portion 17 is so set that a distance T from the inner wall surface of the stabilization portion 17 to the outer circumferential edge of the needle protrusion surface 16a is in a range of 4 to 15 mm. Thereby, it is possible to prevent hindrance of wheal/blister formation due to exertion of a pressure on the wheal/blister from the inner wall surface of the stabilization portion 17.

It suffices that the distance T from the inner wall surface of the stabilization portion 17 to the circumferential edge of the needle protrusion surface 16a is not less than 4 mm, and there is no upper limit for the distance T. However, an increase in the distance T causes an increase in the outer diameter of the stabilization portion 17, which makes it difficult to bring the whole portion of the end face 17a of the stabilization portion 17 into contact with the skin in the case where the needle tube 5 is used to puncture a slender arm, such as an arm of an infant. Taking the slenderness of an infant's arm into account, therefore, it is preferable that the distance T is defined to be 15 mm at maximum.

When the distance S from the circumferential edge of the needle protrusion surface 16a to the outer circumferential surface of the needle tube 5 is not less than 0.3 mm, the adj ustment portion 16 will not enter into the skin. Taking into account the distance T (not less than 4 mm) from the inner wall surface of the stabilization portion 17 to the circumferential edge of the needle protrusion surface 16a and the diameter (about 0.3 mm) of the needle protrusion surface 16a, therefore, the inside diameter d of the stabilization portion 17 can be set to 9 mm or more.

The shape of the stabilization portion 17 is not limited to a cylindrical shape. For example, the shape may be a cylinder with a polygonal sectional shape, such as a tetragonal prism, a hexagonal prism or the like having a cylindrical hole in the center thereof.

The guide portion 18 is a portion of the connection piece 24 which is located on the tip side relative to the stabilization portion 17. The guide portion 18 has a contact surface 18a to be brought into contact with skin. The contact surface 18a is a part of the flat surface 24a of the connection piece 24, and is a flat surface substantially parallel to the end face 17a of the stabilization portion 17. By pressing the stabilization portion 17 against skin until the contact surface 18a of the guide portion 18 makes contact with the skin, a force with which the stabilization portion 17 and the needle tube 5 are pressed against the skin can always be secured to be not less than a predetermined value. Thereby, a portion of the needle tube 5 which protrudes from the needle protrusion surface 16a (a portion corresponding to the protrusion length L) securely punctures the skin.

A distance (hereinafter referred to as "guide portion height") Y from the contact surface 18a of the guide portion 18 to the end face 17a of the stabilization portion 17 is so set that the needle tube 5 and the stabilization portion 17 can be pressed against skin with an appropriate pressure, resulting in appropriate puncture. Thereby, the guide portion 18 guides the pressure exerted on the skin by the needle tube 5 and the stabilization portion 17, the needle tip 5A (the blade surface 5a) of the needle tube 5 can be securely located in the skin upper layer region, and the user can get a feeling of security. The appropriate pressure with which the needle tube 5 and the stabilization portion 17 are pressed against the skin is, for example, 3 to 20 N.

When the inside diameter d of the stabilization portion 17 is in a range of 11 to 14 mm, the guide portion height Y is appropriately determined based on a length (hereinafter referred to as "guide portion length") X from a tip surface of the guide portion 18 to the outer circumferential surface of the stabilization portion 17. For example, when the inside diameter d of the stabilization portion 17 is 12 mm and the guide portion length X is 3.0 mm, the guide portion height Y is set in a range of 2.3 to 6.6 mm.

### [Second Member]

Next, the second member 12 will be described. The second member 12 is formed into a tubular shape. One end portion in the axial direction of the second member 12 is an insertion portion 31 in which the base portion 15 of the first member 11 is to be inserted, and the other end portion is a fitting portion into which the discharge portion of the syringe 103 is to be fitted. A cylindrical hole 31a of the insertion portion 31 is sized correspondingly to the base portion 15 of the first member 11.

A fixation piece 34 is provided on the outer circumferential surface of one endportion in the axial direction of the second member 12 in the insertion portion 31. The fixation piece 34 is formed as a ring-shaped flange projecting radially outward continuously from the distal end of the insertion portion 31. The flat surface 24b of the connection piece 24 provided on the first member 11 comes into contact with and is firmly attached to the fixation piece 34. Examples of a method for firmly attaching the fixation piece 34 and the connection piece 24 to each other include adhesion with an adhesive, ultrasonic fusing, laser fusing, fixation with a fixation screw(s), and the like.

The outer diameter of the fitting portion is set to smaller than the outer diameter of the insertion portion 31. Further, the cylindrical hole of the fitting portion is sized correspondingly to the discharge portion of the syringe 103, and the diameter of the cylindrical hole continuously decreases toward the insertion portion 31. A male screw portion 35 for screwing with the lockmechanism 112 of the syringe 103 is provided on the outer circumferential surface of the fitting portion (see Fig. 1). The elastic member 61 is arranged between the cylindrical hole 31a of the insertion portion 31 and the cylindrical hole of the fitting portion.

### [Elastic Member]

Next, the elasticmember 61 will be described. The elastic member 61 is formed of an elastically deformable member. Examples of the material of the elastic member 61 include elastic materials such as various rubber materials such as natural rubber, silicone rubber, isobutylene rubber, etc., various thermoplastic elastomers based on polyurethane, styrene, or the like, and mixtures thereof.

The elastic member 61 is disposed inside the second member 12, and is interposed between the first member 11 and the syringe 103. The elastic member 61 thus fills up a gap generated between the outer circumferential surface on the proximal end side of the needle tube 5 protruded from the first member 11 and the second member 12. When the discharge portion of the syringe 103 is fitted into the second member 12, the elastic member 61 is elastically deformed, so that the elastic member 61 is closely and liquid-tightly attached to the outer circumference of the needle tube 5. Thereby, it is possible to prevent a drug filled in the syringe 103 from penetrating between the needle tube 5 and the elastic member 61 to leak to the first member 11 side.

### [Lid Member]

Next, the lid member 40 will be described. The lid member 40 is provided on a radially outer edge portion of the guide portion 18 in the needle hub 6. The lid member 40 has a main body portion 41, a connecting portion 42, and an energizing portion 43.

The main body portion 41 is formed into a substantially cylindrical shape. The main body portion 41 has a top plate 44 having a circular shape and a side wall portion 45 that is bent substantially perpendicular from the edge portion of the top plate 44 and is continuously formed along the circumferential direction of the top plate 44.

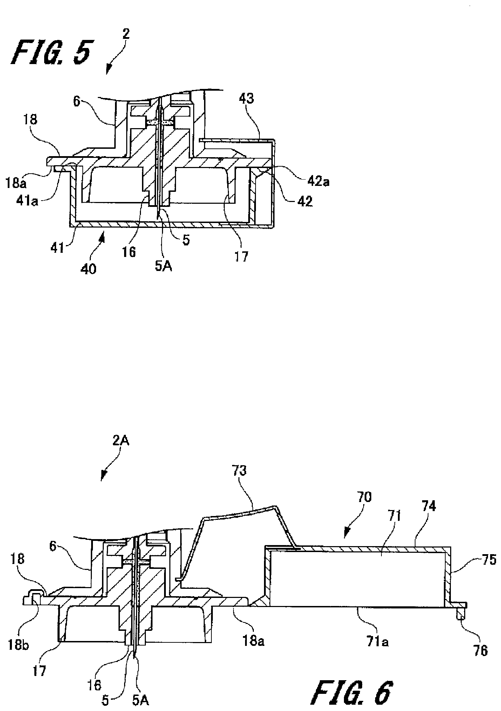

The diameter of the top plate 44 is set to larger than the outer diameter of the stabilization portion 17. The length of the side wall portion 45, that is, the length in the axial direction of the main body portion 41, is set to longer than the guide portion height Y and the length of the needle tube 5 protruded from the adjustment portion 16. In other words, the main body portion 41 is formed into a size that can cover and enclose the whole of the needle tip 5A of the needle tube 5, the adjustment portion 16, and the stabilization portion 17 (see Fig. 5). The main body portion 41 is connected to the needle hub 6 through the connecting portion 42.

The connecting portion 42 is provided to the outer edge portion of the guide portion 18. A portion of the connecting portion 42 which is connected to the guide portion 18 is a thin portion 42a whose thickness is smaller than other portions. The connecting portion 42 rotatably supports the main body portion 41 with the thin portion 42a as a supporting point. The energizing portion 43 is arranged between the connecting portion 42 and the needle hub 6.

The energizing portion 43 is formed by a plate spring having elasticity. The energizing portion 43 includes a plurality of linear portions and bent portions each of which is provided between the linear portions, in Fig. 2 showing a cross section. In the present embodiment, three linear portions and two bent portions are provided. Energization force is accumulated in the energizing portion 43 when, for example, the linear portion is deformed or a bending angle of the bent portion is changed. One end portion in the longitudinal direction of the energizing portion 43 is fixed to the top plate 44 of the main body portion 41. The other end portion in the longitudinal direction of the energizing portion 43 is fixed to the insertion portion 31 of the second member 12 in the needle hub 6.

The portions to which the energizing portion 43 is fixed are not limited to the portions described above. For example, the one end portion in the longitudinal direction of the energizing portion 43 may be fixed to the side wall portion 45 of the main body portion 41 and the other end portion in the longitudinal direction of the energizing portion 43 may be fixed to the fixation piece 34 of the second member 12. That is to say, the one end portion in the longitudinal direction of the energizing portion 43 may be fixed to the main body portion 41 and the other end portion in the longitudinal direction of the energizing portion 43 may be fixed to any piece of the needle hub 6. Further, the energizing portion 43 need not be a portion having linear portions and bent portions, but may be a portion that is curved as a whole (C shape, U shape, and the like) or may be a portion having a combination of these shapes.

In a state before the puncture illustrated in Fig. 2 is performed, the connecting portion 42 and the energizing portion 43 hold the main body portion 41 in a state in which at least a prat of the main body portion 41 protrudes to the distal end side in the axial direction of the needle hub 6 relative to the contact surface 18a of the needle hub 6, that is, to the needle tip 5A side of the needle tube 5. In the present embodiment, in an end portion of the opening side of the main body portion 41, a portion farthest away from the connecting portion 42 protrudes to the distal end side in the axial direction of the needle hub 6 relative to the contact surface 18a. In other words, an end face 41a of the opening side of the main body portion 41, that is, an end face of the side wall portion 45 opposite to the top plate 44, is inclined relative to the contact surface 18a.

In the present embodiment, an example has been described where the main body portion 41 is formed into a cylindrical shape. However, the shape of the main body portion 41 is not limited to this, and for example, the shape may be a cylinder with a polygonal sectional shape, such as a tetragonal prism, a hexagonal prism or the like having a cylindrical hole in the center thereof. Further, the main body portion 41 only has to cover the needle tip 5A of the needle tube 5, so that the main body portion may be formed into a flat plate shape and a part of the needle tip 5A of the needle tube 5 may puncture the main body portion or bend.

1-2. Method of using drug injection device

Next, a method of using the drug injection device 1 having the configuration described above will be described with reference to Figs. 1 to 5.

Fig. 3 is a cross-sectional view illustrating main parts of the drug injection device 1 during puncture. Figs. 4 and 5 are cross-sectional views illustrating main parts of the drug injection device 1 after puncture.

First, as illustrated in Figs. 1 and 2, the syringe 103 is attached to the injection needle assembly 2. Specifically, the discharge portion of the syringe 103 is inserted into the fitting portion of the second member 12 and the lock mechanism 112 is screwed with the male screw portion 35. Thereby, the injection needle assembly 2 has been attached to the syringe 103. As illustrated in Fig. 2, the main body portion 41 of the lid member 40 is away from the needle tip 5A of the needle tube 5, and the needle tip 5A of the needle tube 5 is exposed. The position of the main body portion 41 at this time is defined as a first position.

Next, the end face 17a of the stabilization portion 17 is positioned to oppose the skin. This results in the needle tip 5A of the needle tube 5 being opposed to the skin to be punctured. Next, the drug injection device 1 is moved substantially perpendicularly to the skin to puncture the skin with the needle tip 5A and, simultaneously, press the end face 17a of the stabilization portion 17 against the skin. In this instance, the needle protrusion surface 16a contacts the skin, whereby the skin can be deformed to be flat, and the needle tip 5A side of the needle tube 5 can puncture the skin by the protrusion length L.

Next, the end face 17a of the stabilization portion 17 is pressed against the skin until the contact surface 18a of the guide portion 18 comes into contact with the skin. Here, the guide portion height Y is set to such a value that the skin is punctured while the needle tube 5 and the stabilizationportion 17 are being pressed against the skin with proper pressures. Therefore, the pressure with which the stabilization portion 17 presses the skin is set to a predetermined value.

As a result, the user can recognize the proper pressure relevant to the stabilization portion 17, and can locate the needle tip 5A and the blade surface 5a of the needle tube 5 securely in the skin upper layer region. With the guide portion 18 thus serving as a mark for permitting recognition of the proper pressure relevant to the stabilization portion 17, the user can use the drug injection device 1 without anxiety.

In addition, with the stabilization portion 17 coming into contact with the skin, the posture of the drug injection device 1 is stabilized, and the needle tube 5 can puncture the skin in a straight manner. Further, it is possible to prevent the needle tube 5 from being moved after puncture, so that the drug can be injected stably.

Further, in the case of a needle tube having an extremely small protrusion length of, for example, about 0. 5 mm, the needle tip may fail to pierce skin even when brought into contact with the skin. When the skin pressed by the stabilization portion 17 is pressed down perpendicularly, however, the skin on the inner side of the stabilization portion 17 is pulled, resulting in tension being applied to the skin. Therefore, theskinbecomes less liable to escape from the needle tip 5A of the needle tube 5. Accordingly, it is also possible to obtain an effect that the needle tip 5A more easily punctures the skin by providing the stabilization portion 17.

At least a part of the main body portion 41 of the lid member 40 protrudes to the distal end side in the axial direction of the needle hub 6 relative to the contact surface 18a. Therefore, the end face 41a of the main body portion 41 comes into contact with the skin before the contact surface 18a comes into contact with the skin. As described above, by pressing the injection needle assembly 2 until the contact surface 18a comes into contact with the skin, the main body portion 41 rotates in a direction in which the main body portion 41 goes away from the needle tip 5A of the needle tube 5 with the thin portion 42a of the connecting portion 42 as a supporting point. As a result, the end face 41a of the main body portion 41 becomes parallel with the contact surface 18a.

When the main body portion 41 rotates in a direction in which the main body portion 41 goes away from the needle tip 5A of the needle tube 5, the energizing portion 43 is elastically deformed, so that a linear portion in the energizing portion 43 may be deformed or a bending angle of a bent portion may be changed. Therefore, the energization force is accumulated in the energizing portion 43.

After puncturing the skin with the needle tip 5A of the needle tube 5, the gasket 113 is moved toward the discharge portion by pressing the pusher member 104 (see Fig. 1). Thereby, the drug filled in the liquid chamber 114 of the syringe 103 is pushed out from the discharge portion and injected into the skin upper layer region from the needle tip 5A through the needle hole of the needle tube 5. In this instance, no space is formed between the distal end of the discharge portion and the proximal end of the needle tube 5, so that it is possible to reduce the remaining amount of the drug.

Next, as illustrated in Fig. 4, the drug injection device 1 is separated from the skin to cause the end face 17a of the stabilization portion 17, the needle protrusion surface 16a, and the end face 41a of the main body portion 41 to go away from the skin. In this instance, the pressure from the skin to the energizing portion 43 through the main body portion 41 is released. Then, the main body portion 41 is pressed by a restoring force (energization force) of the energizing portion 43 to rotate in a direction to approach the needle tip 5A of the needle tube 5 with the thin portion 42a of the connecting portion 42 as a supporting point.

Then, as illustrated in Fig. 5, the end face 41a of the main body portion 41 comes into contact with or approaches the contact surface 18a, so that the rotation action of the main body portion 41 stops. Thereby, the needle tip 5A of the needle tube 5, the adjustment portion 16, and the stabilization portion 17 are covered and enclosed by the main body portion 41. The position where the main body portion 41 most approaches the needle tip 5A of the needle tube 5 is defined as a second position. Then, a finger or the like is prevented from touching the needle tip 5A of the needle tube 5 after puncture by the main body portion 41.

As a result, it is possible to keep the needle tip 5A of the needle tube 5 that has been used in a safe state and it is possible to prevent the used needle tip 5A of the needle tube 5 from puncturing a user against the intention of the user. Further, the used needle tip 5A of the needle tube 5 is covered by the main body portion 41, so that it is possible to prevent blood attached to the needle tip 5A from scattering, and it is also possible to prevent blood-borne infection.

Regarding the lid member 40 of the present embodiment, when the drug injection device 1 is separated from the skin, the main body portion 41 is automatically rotated by the energization force accumulated in the energizing portion 43. As a result, it is possible to prevent omission of closing of the main body portion 41, and it is also possible to prevent a finger or the like from erroneously touching the needle tip 5A of the needle tube 5 when rotating the main body portion 41.

After the puncture, the energizing portion 43 of the rotated lid member 40 energizes the main body portion 41 in a direction in which the end face 41a of the main body portion 41 is pressed against the contact surface 18a. Thereby, it is possible to prevent the main body portion 41 from rotating in a direction in which the main body portion 41 goes away from the needle tip 5A of the needle tube 5 against the intention of the user and prevent the needle tip 5A of the needle tube 5 from being exposed.

### 2. Second Embodiment

Next, a drug injection device according to the second embodiment will be described with reference to Fig. 6.

Fig. 6 is an enlarged cross-sectional view illustrating main parts of the drug injection device according to the second embodiment.

The difference between the drug injection device according to the second embodiment and the drug injection device 1 according to the first embodiment is a configuration of the lid member in the injection needle assembly. Therefore, here, the lid member will be mainly described, and the same portions as those of the injection needle assembly 2 according to the first embodiment will be denoted by the same reference numerals and redundant description will be omitted.

As illustrated in Fig. 6, the injection needle assembly 2A has the hollow needle tube 5, the needle hub 6 that holds the needle tube 5, and a lid member 70.

The lid member 70 has a main body portion 71 having a substantially cylindrical shape, a connecting portion 72, and an energizing portion 73. The main body portion 71 has a top plate 74 having a circular shape, a side wall portion 75, and a contact protrusion 76.

The contact protrusion 76 is arranged at a position farthest away from the connecting portion 72 on an end face 71a of the main body portion 71. The contact protrusion 76 protrudes from the end face 71a in a direction going away from the top plate 74.

The main body portion 71 is rotatably supported by the connecting portion 72. In a state before the puncture is performed, the connecting portion 72 and the energizing portion 73 hold the main body portion 71 so that the end face 71a of the main body portion 71 becomes substantially in parallel with the contact surface 18a. Therefore, the contact protrusion 76 protrudes to the distal end side in the axial direction of the needle hub 6 relative to the contact surface 18a.

When the drug injection device having such a lid member 70 is pressed against the skin, the contact protrusion 76 of the main body portion 71 comes into contact with the skin before the contact surface 18a comes into contact with the skin. When the injection needle assembly 2A is pressed until the contact surface 18a comes into contact with the skin, the main body portion 71 rotates in a direction in which the main body portion 71 goes away from the needle tip 5A of the needle tube 5 with a thin portion 72a of the connecting portion 72 as a supporting point. As a result, the energizing portion 73 is elastically deformed and the energization force is accumulated in the energizing portion 73.

A fitting recessed portion 18b into which the contact protrusion 76 of the rotated lid member 70 is fitted is provided in the contact surface 18a of the guide portion 18. Thereby, it is possible to prevent the lid member 70 that has rotated to cover and enclose the needle tip 5A of the needle tube 5, the adjustment portion 16, and the stabilization portion 17 from rotating again.

The other portions are the same as those of the injection needle assembly 2 according to the first embodiment, so that the description thereof will be omitted. When the lid member 70 having the configuration as described above is provided to the injection needle assembly 2A, it is possible to obtain the same functional effect as that of the injection needle assembly 2 according to the first embodiment described above.

### 3. Third Embodiment

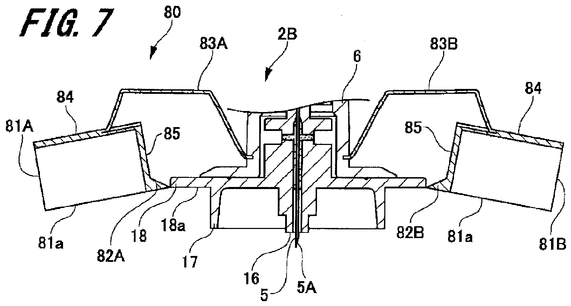

Next, a drug injection device according to the third embodiment will be described with reference to Fig. 7.

Fig. 7 is an enlarged cross-sectional view illustrating main parts of the drug injection device according to the third embodiment.

The difference between the drug injection device according to the third embodiment and the drug injection device 1 according to the first embodiment is a configuration of the lid member in the injection needle assembly. Therefore, here, the lid member will be mainly described, and the same portions as those of the injection needle assembly 2 according to the first embodiment will be denoted by the same reference numerals and redundant description will be omitted.

As illustrated in Fig. 7, the injection needle assembly 2B has the hollow needle tube 5, the needle hub 6 that holds the needle tube 5, and a lid member 80.

The lid member 80 has a first main body portion 81A, a first connecting portion 82A, a first energizing portion 83A, a second main body portion 81B, a second connecting portion 82B, and a second energizing portion 83B. The first main body portion 81A, the first connecting portion 82A, and the first energizing portion 83A are provided to an outer edge portion of the guide portion 18. The second main body portion 81B, the second connecting portion 82B, and the second energizing portion 83B are provided to an outer edge portion of the guide portion 18 opposite to the first main body portion 81A, the first connecting portion 82A, and the first energizing portion 83A with the guide portion 18 in between.

The configuration of the second main body portion 81B, the second connecting portion 82B, and the second energizing portion 83B is the same as that of the first main body portion 81A, the first connecting portion 82A, and the first energizing portion 83A. Therefore, here, the first main body portion 81A, the first connecting portion 82A, and the first energizing portion 83A will be described.

The first main body portion 81A is formed into a substantially semi-cylindrical shape. The first main body portion 81A has a substantially semicircular top plate 84 and a side wall portion 85. The first main body portion 81A is rotatably supported by the first connecting portion 82A. In a state before the puncture is performed, the first connecting portion 82A and the first energizing portion 83A hold the first main body portion 81A so that at least a part of the first main body portion 81A protrudes to the distal end side in the axial direction of the needle hub 6 relative to the contact surface 18a.

After the puncture, the lidmember 80 rotates in a direction in which the first main body portion 81A and the second main body portion 81B approach the needle tip 5A of the needle tube 5. Then, the needle tip 5A of the needle tube 5 is covered and enclosed by the first main body portion 81A and the second main body portion 81B.

The other portions are the same as those of the injection needle assembly 2 according to the first embodiment, so that the description thereof will be omitted. When the lid member 80 having the configuration as described above is provided to the injection needle assembly 2B, it is possible to obtain the same functional effect as that of the injection needle assembly 2 according to the first embodiment described above.

According to the lid member 80 of the third embodiment, the main body portion is divided into two portions, which are the first main body portion 81A and the second main body portion 81B. Even when the needle tube 5 is used to puncture a slender arm, such as an arm of an infant, it is possible to reliably press either of the first main body portion 81A or the second main body portion 81B against the skin. As a result, after the puncture, at least either of the first main body portion 81A or the second main body portion 81B can be rotated, so that it is possible to reliably cover the needle tip 5A of the needle tube 5.

The embodiments of the drug injection device and the injection needle assembly and also the functional effects thereof have been described. However, the drug injection device and the injection needle assembly of the present invention are not limited to the embodiments described above, but various modifications are possible without departing from the scope of the invention set forth in the claims.

In the embodiments described above, an example is described where the luer lock unit is provided as the lock mechanism 112, but this is not limitative. The discharge portion and the injection needle assembly may be screwed together by providing a male screw portion to the discharge portion and providing a female screw portion to the cylindrical hole of the secondmember 12 of the injection needle assembly.

### Reference Signs List

1: drug injection device, 2, 2A, 2B: injection needle assembly, 5: needle tube, 5A: needle tip, 5a: blade surface, 6: needle hub, 11: first member, 12: second member, 13: gasket, 15: base portion, 16: adjustment portion, 16a: needle protrusion surface, 17: stabilization portion, 17a: end face, 18: guide portion, 18a: contact surface, 18b: fitting recessed portion, 40, 70, 80: lid member, 41, 71: main body portion, 41a, 71a: end face, 42, 72: connecting portion, 42a, 72a: thin portion, 43, 73: energizing portion, 44, 74, 84: top plate, 45, 75, 85: side wall portion, 76: contact protrusion, 81A: first main body portion, 81B: second main body portion, 82A: first connecting portion, 82B: second connecting portion, 83A: first energizing portion, 83B: second energizing portion, 103: syringe, 104: pusher member, 105: syringe holder, 105a: engaging portion, 111 : syringe main body, 112: lock mechanism, 113: gasket, 114: liquid chamber, 115: flange portion, 116: plunger main body, 117: operation portion, 118: view window, 119: holder brim portion

## Claims

1. An injection needle assembly comprising:
a needle tube including a needle tip that can puncture a living body;
a needle hub that holds the needle tube and has a contact surface that comes into contact with a living body when the needle tip of the needle tube punctures the living body;
a main body portion that can move to a first position which is away from the needle tip of the needle tube and where the needle tip of the needle tube is exposed and a second position where the main body portion covers the needle tip of the needle tube;
a connecting portion that rotatably connects the main body portion to the needle hub; and
an energizing portion that is provided to the main body portion and energizes the main body portion from the first position toward the second position when being elastically deformed,
wherein, in the first position, at least a part of the main body portion protrudes to the needle tip side of the needle tube relative to the contact surface.

2. The injection needle assembly according to claim 1, wherein
the main body portion has an end face that comes into contact with or approaches the contact surface in the second position, and
the end face is inclined toward the needle tip side of the needle tube relative to the contact surface in the first position.

3. The injection needle assembly according to claim 1 or 2, wherein
the main body portion has a contact protrusion that protrudes toward the needle tip side of the needle tube relative to the contact surface in the first position.

4. The injection needle assembly according to any one of claims 1 to 3, wherein
the contact surface is one surface of a guide portion that guides pressure of the needle tube to a living body by being contacted with skin when the needle tube punctures the living body.

5. The injection needle assembly according to any one of claims 1 to 4, wherein
the energizing portion includes a plate spring having elasticity, and
one end of the energizing portion is fixed to the main body portion and the other end of the energizing portion is fixed to the needle hub.

6. A drug injection device comprising:
an injection needle assembly including a needle tube having a needle tip that can puncture a living body; and
a syringe that is detachably attached to the injection needle assembly,
wherein the injection needle assembly includes
a needle hub that holds the needle tube and has a contact surface that comes into contact with a living body when the needle tip of the needle tube punctures the living body,
a main body portion that can move to a first position which is away from the needle tip of the needle tube and where the needle tip of the needle tube is exposed and a second position where the main body portion covers the needle tip of the needle tube,
a connecting portion that rotatably connects the main body portion to the needle hub, and
an energizing portion that is provided to the main body portion and energizes the main body portion from the first position toward the second position when being elastically deformed, and
in the first position, at least a part of the main body portion protrudes to the needle tip side of the needle tube relative to the contact surface.
